# EUROPEAN PATENT SPECIFICATION

(11) **EP 1 139 743 B1**
(45) Date of publication and mention of the grant of the patent: **22.03.2006**
(21) Application number: 99967401.3
(22) Date of filing: 16.12.1999
(51) Int. Cl.: A01N 33/18, A01N 33/24, A61K 31/13, A61K 31/015, C07C 209/88, C07D 217/26

(54) **EXO-S-MECAMYLAMINE FORMULATION AND USE IN TREATMENT**
EXO-S-MECAMYLAMIN-FORMULIERUNG UND IHRE VERWENDUNG IN BEHANDLUNGEN
FORMULATION A BASE D'EXO-S-MECAMYLAMINE ET UTILISATION EN TRAITEMENT

(30) Priority: 16.12.1998 US 112534 P
(43) Date of publication of application: 10.10.2001
(60) Divisional application: 05024899.6
(73) Proprietor: UNIVERSITY OF SOUTH FLORIDA, Tampa, FL 33620-7900 (US)
(72) Inventor: SHYTLE, Douglas, Lutz, FL 33549 (US); SANBERG, Paul, Spring Hill, FL 34610 (US); NEWMAN, Mary, Valrico, FL 33954 (US); SILVER, Archie, Tampa, FL 33624 (US)
(74) Representative: Roques, Sarah Elizabeth
(86) International application number: PCT/US1999/030153
(87) International publication number: WO 2000/035279

(56) References cited:
- US-A- 5 039 801
- SUCHOCKI, JOHN A. ET AL: "Synthesis of 2-exo- and 2-endo-mecamylamine analogs. Structure-activity relationships for nicotinic antagonism in the central nervous system" JOURNAL OF MEDICINAL CHEMISTRY (1991), 34(3), 1003-10 , 1991, XP002218706

## Description

### Technical Field

The present invention is in the field of chemical synthesis of stereoisomers and more particularly the exo-S-mecamylamine enantiomer and use in medical treatments.

### Background Art

Mecamylamine (N,2,3,3-tetramethylbicyclo-[2.1.1]heptan-2-amine hydrochloride, 826-39-1) was developed and characterized by Merck & Co., Inc., as a ganglionic blocker with clinically significant hypotensive actions (Stone et al., J Med Pharm Chem 5(4);665-90, 1962). Unique characteristics of mecamylamine - including exceptional oral efficacy, rapid onset, long duration of action, and nearly complete absorption from the gastrointestinal tract - made mecamylamine at that time more desirable than the existing ganglionic blockers (Baer et al., 1956).

Despite mecamylamine's proven efficacy in the treatment of hypertension, its side effects resulting from broad parasympathetic inhibition led to its demise as a first line treatment for essential hypertension. Generalized ganglionic blockade may result in atony of the bladder and gastrointestinal tract, impaired sexual function, cycloplegia, xerostomia, diminished perspiration and postural hypotension. Among mecamylamine side effects experienced at the antihypertensive dose of 25 mg/day were cardiovascular effects, hypothermia, tremors, anti-diuresis, antinociception, blurred vision, impotency, dysuria, tremor, choreiform movements, mental aberrations, nervousness, depression, anxiety, insomnia, slurred speech, weakness, fatigue, sedation, headache, constipation and renal insufficiency. Even at lower doses, such as 7.5 mg/day, some evidence for constipation has been reported. Minor increases in taste perversion (altered sense of taste), dizziness, insomnia and dyspepsia were noted. Mecamylamine continued to be used in special situations, such as hypertensive encephalopathy (Moser, 1969), hypertensive crises, and autonomic dysreflexia (Badddom and Johnson, 1969; Braddom and Rocco, 1991). Outside of a few laboratories and an occasional clinical study, sales of mecamylamine are rare.

In addition to its peripheral ganglionic blocking actions, mecamylamine crosses the blood brain barrier and functions as a selective nicotinic receptor antagonist at doses which do not have a significant effect on parasympathetic function (Banerjee et al., 1990; Martin et al.,-1993). As a result, mecamylamine blocks most of the physiological, behavioral, and reinforcing effects of tobacco and nicotine (Martin et al., 1989). In studies of nicotine dependence, doses of 2.5 to 20 mg have been administered acutely to human subjects. For example, Rose et al. (1989) found that low doses of mecamylamine (2.5 to 10 mg), which were well tolerated, reduced the subjective effects of smoking in adult smokers.

In a recent double blind placebo-controlled study investigating the benefits of oral mecamylamine (5 mg/day b.i.d.) in adults for smoking cessation treatment, there was no significant increase over controls in adverse effects reported with mecamylamine treatment for most symptoms, including blurred vision, dizziness when standing, dry mouth, weakness, abdominal pains, or difficult urination. The most prevalent symptom with the mecamylamine treatment was mild constipation; at some point during the five weeks of mecamylamine treatment, 70% of the subjects reported that symptom versus 32% in the placebo group (Rose et al., 1994). Mecamylamine also has been reported to alter cognitive functioning (Newhouse PA et al, Neuropsychopharmacology 10: 93-107, 1994), electrical brain waves (Pickworth WB, Herning RI, Henningfield JE, Pharmacology Biochemistry & Behavior 30: 149-153, 1988) and cortical blood flow (Gitalman DR, Prohovnik I, Neurobiology of Aging 13: 313-318, 1992).

While most animal studies used more than 0.5 mg/kg, Driscoll found that a small dose of only mecamylamine (<0.3 mg/kg, not 0.5 mg/kg) to high-avoidance rats increased their avoidance success almost as much as 0.1 mg/kg nicotine (but less than 0.2 mg/kg nicotine). Based on his experiments, Driscoll concluded: "mecamylamine may exert unpredictable effects on rats at the dosage levels used to block nicotine in behavioral tests" (Driscoll P., Psychophannacologia (Berl.) 46:119-21, 1976).

Many organic compounds exist in optically active forms, i.e., they have the ability to rotate the plane of plane-polarized light. In describing an optically active compound, the prefixes R and S are used to denote the absolute configuration of the molecule about its chiral center(s). The prefixes (+) and (-) or *d* and *l* are employed to designate the sign of rotation of plane-polarized light by the compound, with (-) and *l* meaning that the compound is levorotatory. A compound prefixed with (+) and *d* is dextrorotatory. For a given chemical structure, these compounds, called stereoisomers, are identical except that they are mirror images of one another. A specific stereoisomer may also be referred to as an enantiomer, and a mixture of such isomers is often called an enantiomeric or racemic mixture.

Stereochemical purity is of importance in the field of pharmaceuticals, where 12 of the 20 most prescribed drugs are optically active. One example is the *l*-form of propranolol, which, is about 100 times more potent than the *d*-form. Optical purity is important since certain isomers may be deleterious rather than simply inert. Another example is *d*-thalidomide that appears to be a safe and effective sedative for controlling morning sickness during pregnancy; whereas, *l-*thalidomide is thought to be a potent teratogen.

Mecarnylamine has been marketed as a racemic mixture comprising the optical isomers exo-S-mecamylamine and exo-R-mecamylamine hydrochloride. Previous studies aimed at investigating the pharmacology of these two isomers have generally found little or no difference in potency or efficacy. For example, Stone et al. (1962) compared the effects of(+)-mecamylamine hydrochloride with racemic mecamylamine hydrochloride on nicotine-induced convulsions and pupil dilation and found essentially no significant differences between the two compounds and concluded that "optical isomerism does not play a significant role in determining the degree of activity." (Stone, supra, p.-675.). Schonenberger et al. (1986) reported "interesting differences" in the actions of *d-* and *l*-mer-amylamine hydrochloride in assays measuring neuromuscular transmission. However, they provided no details on the differences.

In U.S. Patent No. 5,039.801. Brossi and Schonenberger disclosed that "the antipodes (-)-and (+)-mecarnylamine were obtained here from the corresponding methylbenzylureas in 40% yield each and were of high optical purity (95%, HPLC), affording hydrochloride salts which were optically pure after one crystallization." (col. 3, lines 32-37) However, in disclosing their experimental findings, they mention that the "etheral extract of the concentrated, acidified reaction mixture was concentrated and the residue distilled (Kugel, 180°, 20 torr) to give 6.08. g (96%) (-)-12 as a tlc. pure colorless liquid which turned to a waxy solid on standing in cold: [α]_{D}= -77.0° (c+2.6 in benzene) lit. (+)-12: [α]_{D}= +80.1° (c=3 in benzene). The combined org extracts from the alkaline aqueous phase were concentrated, the resulting liquid was mixed with 20 ml Et₂O and crude hydrochloride (+)-1.HCl was precipitated by addition of a slight excess of HCl in Et₂O. After filtration, the finely powdered colorless solid was recrystallized from 2-propanol to give 1.02 g (64%) (+)-1.HCl as needles [A]_{D}+20.1° (c+1.7 in CHCl₃). The more polar urea 3 (1.85 g, 5.89 mmol) was treated in exactly the same manner to give 752 mg (63% (-)-1.HCl as colorless needles: [A]_{D}-20.0° (c=2.2 in CNCl₃)." Col. 6, lines 20-37. However, no in vitro or in vivo data were disclosed.

Suchocki et al. (1991) J. Med. Chem. Vol 34, No 3 pp 1003-1010 investigated the actions of *d*- and *l*-mecamylamine hydrochloride in assays measuring nicotine-induced depression of spontaneous locomotor activity and antinociception. They found that both optical isomers had similar potency in blocking the antinociception caused by nicotine; whereas, the potency of the (+)-mecamylamine isomer in blocking the nicotine-induced depression of spontaneous locomotor activity was unable to be determined due to an experimental confound.

Tourette's syndrome (TS) is an autosomal dominant neuropsychiatric disorder characterized by a range of symptoms, including multiple motor and phonic tics. It is a hyperkinetic movement disorder expressed largely by sudden, rapid, brief, recurrent, nonrhythmic, stereotyped motor movements (motor tics) or sounds (phonic tics), experienced as irresistible impulses but which can be suppressed for varying lengths of time (Tourette Syndrome Classification Study Group, Arch Neurol 50: 1013-16). Motor tics generally include eye blinking, head jerking, shoulder shrugging and facial grimacing, while phonic or vocal tics include throat clearing, sniffling, yelping, tongue clicking and coprolalia. The symptoms typically begin in childhood and range from relatively mild to very severe over the course of a patient's lifetime (Robertson MM, Br J Psychiatry, 154:147-169, 1989). Many TS patients also exhibit other neuropsychiatric abnormalities including obsessive compulsive symptoms (Pauls DL et al. Psychopharm Bull, 22:730-733, 1986), hyperactivity and attention deficit (Comings DE, Himes JA, Comings BG, J Clin Psychiatry, 51:463-469, 1990). Problems with extreme temper or aggressive behavior also are frequent (Riddle MA et al. Wiley Series in Child and Adolescent Mental Health, Eds. Cohen DJ, Bruun, RD, Leckman JF, New York City, John Wiley and Sons, pp. 151-162, 1988; Stelf ME, Bornstein RA, Hammond L, A survey of Tourette syndrome patients and their families: the 1987 Ohio Tourette Survey, Cincinnati, Ohio Tourette Syndrome Association, 1988), as are school refusal and learning disabilities (Harris D, Silver AA, Learning Disabilities, 6(1):1-7, 1995; Silver AA, Hagin RA, Disorders of Learning Childhood, Noshpitz JD, ed. New York City: Wiley, pp. 469-508, 1990).

While the pathogenesis of TS is still unknown, excessive striatal dopamine and/or dopamine receptor hypersensitivity has been proposed (Singer HS et al. Ann Neurol, 12:361-366, 1982), based largely on the therapeutic effectiveness of dopamine receptor antagonists. TS is frequently treated with the dopamine antagonist haloperidol (Haldol®, McNeil Pharmaceutical, Raritan, NJ), which is effective in about 70% of cases (Erenberg G, Cruse RP, Rothner, AD, Ann Neurol, 22:383-385, 1987; Shapiro AK, Shapiro E, Wiley series in child and adolescent mental health, Eds. Cohen DJ, Bruun RD, Leckman JF, New York City, John Wiley and Sons, pp. 267-280, 1988). Other neuroleptics include pimozide (Shapiro ES et al. Arch Gen Psychiatry, 46:722-730, 1989), fluphenazine (Singer HS, Gammon K, Quaskey S. Pediat Neuroscience, 12:71-74, 1985-1986), and risperidone (Stamenkovic et al., Lancet 344:1577-78, 1994). The α-adrenergic agonist clonidine, which also is effective for associated attention deficit hyperactivity disorder (ADHD), has only a 40% success rate for motor and vocal tics (Bruun RD, J Am Acad Child Psychiatry, 23: 126-133, 1984; Cohen DJ et al. Arch Gen Psychiatry 37: 1350-1357, 1980). Other medications with varying degrees of effectiveness include clonazepam (Gonce M, Barbeau A. Can J Neurol Sci 4: 279-283, 1977), naloxone (Davidson PW et al. Appl Res Ment Retardation 4: 1-4, 1983) and fluoxetine (Riddle MA et at. J Am Acad Child Adol Psychiatry 29: 45-48, 1990). A commonly used medication is haloperidol (Erenberg G, Cruse RP, Rothner AD, Ann Neurol, 22:383-385, 1987). However, therapeutic doses of haloperidol frequently cause difficulty in concentration, drowsiness, depression, weight gain, parkinsonian-like symptoms - and with long-term use - tardive dyskinesia (Shapiro AK, Shapiro E, Tourette's syndrome and Tic Disorders: Clinical Understanding and Treatment. Wiley series in child and adolescent mental health. Eds. Cohen, DJ, Bruun, RD, Leckman JF, New York City, John Wiley and Sons, pp. 267-298, 1988). The side effect of tardive dyskinesia is particularly bothersome because it may add additional abnormal, involuntary movements of the tongue, jaw, trunk and/or extremities.

Erenberg et al. (Erenberg G, Cruse RP, Rothner AD, Ann Neurol 22:383-385, 1987) found that most patients with TS stop using their haloperidol or other neuroleptic medications by age 16, often because of side effects. After TS patients quit medication, they have less control over speech and movement, which disqualify many for full-time, responsible jobs. The public, including law enforcement officers, often identify the symptoms as intoxication. Unexpected movements and coprolalia cause great social difficulties.

It has been observed that 50% of children presenting with TS also have Attention Deficit Hyperactivity Disorder (ADHD). ADHD is a neurobiological disorder characterized by impaired attentiveness, increased impulsivity, and hyperactivity. ADHD is now the most commonly diagnosed childhood psychiatric condition, with some 3.5 million afflicted. In addition, 60% of adolescents with ADHD continue to have symptoms in adulthood, representing another 2.5 million patients.

Many neuropsychiatric disorders involve abnormal or involuntary movements including but not limited to obsessive-compulsive disorder (OCD), TS, ADHD, hemidystonia, and Huntington's disease. These diseases may be caused by neurochemical imbalances in the brain's basal ganglia. Acetylcholine, by activating nAChrs in the basal ganglia, regulates motor activity in humans (Clarke PBS, Pert A, Brain Res 348: 355-358, 1985). Nicotinic stimulation excites activity in the dopamine (DA)-producing cells in the basal ganglia (Clarke PBS et al, J Pharmacol Exper Therapeutics 246: 701-708, 1988; Grenhoff J, Aston-Jones G, Svennson TH, Acta Physiol Scand 128: 351-358, 1986 ; Imperato A, Mulas A, Di Chiara G, Eur J Pharmacol 132: 337-338,1986), while mecamylamine blocks nAChr and inhibits DA release from basal ganglia structures (Ahtee L, Kaakkola S, Br J Pharmacol 62: 213-218,1978).

U. S. Patent No. 5,774,052 to Rose and Levin discloses agonist-antagonist combinations to reduce the use of nicotine and other drugs. In combination with nicotine, the nicotinic antagonist mecamylamine was given to treat tobacco dependency. Rose and Levin proposed including both nicotine and mecamylamine in a patch. Rose and Levin also suggested that such agonist-antagonist combinations could be used in other psychopathological disorders and cases involving neuronal dysfunction (e. g., manic depression, schizophrenia and hypertension due to sympathetic autonomic disorder).

It would benefit patients to be able to have better symptom control and fewer side effects. Our clinical experience with mecamylamine racemate in human patients with a variety of disorders supports a variety of uses. Herein is disclosed improved symptom control with exo-R-mecamylamine for the treatment of a variety of nicotine-responsive neuropsychiatric disorders.

### Disclosure of Invention

The invention provides the use of exo-S-mecamylamine or a pharmaceutically acceptable salt thereof, substantially free of exo-R-mecamylamine in the manufacture of a medicament for the treatment of neuropsychiatric disorders in a human. Preferably the amount of said exo-S-mecamylamine or salt is about 0.5 mg to about 20 mg. A preferred medicament contains exo-S-mecamylamine hydrochloride and a pharmaceutically acceptable carrier. The medicament can be adapted for oral, intravenous administration. The medicament can be a transdermal patch, solid preparation, or a sustained release form. Preferably, the substantially pure exo-S-mecamylamine is greater than 95% by weight and exo-R-mecamylamine is less than 5% by weight. More preferably, the substantially pure exo-S-mecamylamine is greater than greater than 99% by weight and exo-R-mecamylamine is less than 1% by weight. Even more preferably, the substantially pure exo-S-mecamylamine is greater than 99.5% by weight and exo-R-mecamylamine is less than 0.5% by weight. Most preferably, the substantially pure exo-S-mecamylamine is greater than 99.7% by weight and exo-R-mecamylamine is less than 0.3% by weight.

Described herein are treatments of medical conditions by administering a therapeutically effective amount of exo-S-mecamylamine or a pharmaceutically acceptable salt thereof, substantially free of its exo-R-mecamylamine, said amount being sufficient to ameliorate the medical condition. Preferably, the method provides for administering exo-S-mecamylamine intravenously, transdermally, intrathecally, orally or by bolus injection. Preferably, the dosage of exo-S-mecamylamine is about 0.5 mg to about 20 mg. Preferably, exo-S-mecamylamine is administered one to four times per day. The medical conditions include but are not limited to substance addiction (involving cocaine, alcohol, amphetamine, opiate, other psychostimulant and a combination thereof), Tourette's Syndrome and other tremors, bipolar disorder, depression, an anxiety disorder, schizophrenia, a seizure disorder, Parkinson's disease, attention deficit hyperactivity disorder, obsessive compulsive disorder, hemidystonia and Huntingdon's disease.

Also described herein is a method for eliciting an anti-nicotine effect that is of longer duration than the effect of a comparable dose of racemic mecamylamine This method includes administering to an individual in need thereof an amount of exo-S-mecamylamine that produces an anti-nicotine effect of more than twice the duration of racemic mecamylamine, the exo-S-mecamylamine containing at least 90% by weight of exo-S-mecamylamine and less than about 10% by weight of exo-R-mecamylamine.

### Brief Description Of Drawings

Figure 1 is a gas chromatograph printout showing that exo-S-mecamylamine elutes purely at 63.971 minutes after placement on the column.
Figure 2 shows the structures of mecamylamine generally (+/-), exo-R-mecamylamine and exo-S-mecamylamine.
Figure 3 is a graph showing total distance traveled in 60 minutes by rats having undergone seven days of sensitization with saline or mecamylamine at one of 3 doses. The dagger symbol indicates significant differences from the Sal/Sal group. The asterisk identifies significant differences from the Sal/Nic group.
Figure 4 is a graph showing the center distance traveled by rats in the same study.
Figure 5 is a graph showing the vertical activity of rats in the same study.
Figure 6 is a bar graph displaying ambulatory behavior among treatment and control groups.
Figure 7 is a bar graph shows rearing behavior among the treatment and control groups.
Figure 8 is a bar graph showing stereotypic behavioral counts among the control and treated groups.
Figure 9 is a bar graph showing mean center distance traveled following injection with saline or a form of mecamylamine.
Figure 10 is a bar graph showing locomotor response (total distance) to nicotine alone 24 hours before and 24 hours after two-day treatment with mecamylamine/nicotine combination.
Figures 11A and 11B show the effects of forms of mecamylamine on haloperidol-induced catalepsy. Figure 11A shows mean values; Figure 11B shows median values.

### Best Mode For Carrying Out The Invention

Although there is some variability from one patient to another, it is generally observed that, by administering an effect amount of only exo-R-mecamylamine, it is possible to accomplish a more "targeted" therapy, which provides the desired effect without the consequences of all the other pharmacologic effects. This is important since it is not desirable for all patients to be administered a compound with such a multifaceted spectrum of activity.

Synthesis of mecamylamine has been disclosed in three patents: U.S. Patent Nos. 2,831,027 (1958), 2,885,428 (1959) and 5,986,142 (1999).

For the synthesis of mecamylamine one starting material is camphene, the racemate or either enantiomer. The enantiomers are available from natural sources or are can be obtained by resolution using liquid chromatography using a chiral medium (Armstrong, J Chrom A, 666: 445, 1994). They can also be made using kinetic resolution wherein a chiral reagent selectively reacts with one enantiomer leaving the other intact (Jenke, J Organomet Chem, 405: 383, 1991). The camphene enantiomers can also be made from chiral precursors (Hana, Chem Ber, 111: 2527, 1978).

Camphene, racemic or enantiomeric, in an acidic medium can be reacted with a nitrogen source, such as azide (Pancrazi, Bull Chim Soc (Fr.), (1977) 162), cyanide (Stein, J Am Chem Soc,78: 1514, 1956; Stone, J Med Pharm Chem, 5: 665, 1962; Pfister, US Patent No 2,831,027 (1958)) or thiocyanate (Luskin, US Patent No 2,885,428; CA. 53:20124h). The intermediates so produced can be converted to mecamylamine, the racemate or either enantiomer.

Camphene, racemic or enantiomeric, can be converted to camphene hydrochloride (Gream, Aust J Chem, 27: 567, 1974) which can be reacted with nitrite (Huckel, Ann 528 (1937) 57; CA. 31:3033-4) to produce an intermediate which can be converted to mecamylamine, the racemate or either enantiomer. The hydrochloride can also be reacted with an amine to yield mecamylamine, racemic or enantiomeric (Stone, J Med Pharm Chem, 5: 665, 1962), or an intermediate that can be converted to mecamylamine, racemic or either enantiomer.

Camphenilone, racemic or as either of its enantiomers, can be reacted with a methyl lithium or similar nucleophilic methyl to give an alcohol (Stone, J Med Pharm Chem, 5: 665, 1962; Gream, Aust J Chem, 27 (1974) 567). The alcohol or its derivatives can be subjected to the acidic reactions described above for camphene to yield mecamylamine, racemic or as either of its enantiomers, or products which can be converted to it. (Stone, J Med Pharm Chem, 5: 665, 1962). A similar alcohol can be made from camphene, racemic or enantiomeric, (Coxon, Tetrahedron, 26: 3755, 1970) and subjected to the same reactions yielding similar products.

The reaction of organic azides with camphene, racemic or as either of its enantiomers followed by either photolytic or thermal decomposition (Huisgen, Chem Ber, 98: 3992, 1965; Franz, J Org Chem, 29: 2922, 1964) of the reaction product yields an aziridine which can be ring opened (Gold, J Org Chem, 37: 2208; 1972) and transformed into mecamylamine, the racemate or either enantiomer.

Mecamylamine can be synthesized in either the racemic form or the enantiomers. The racemic product can be resolved into its enantiomers by salt formation using chiral acids (carboxylic, sulphonic, phosphoric (Pfister, US Patent No 2,831,027 (1958); Stone, J Med Pharm Chem, 5: 665, 1962) and then the enantiomer regenerated, by derivatization with chiral molecules. The resulting diastereomers can be separated by crystallization or by simple chromatography (Schonenberger, Helv.Chim. Acta., 69 (1986) 283.), and then the enantiomer regenerated, or by liquid chromatography using a chiral medium.

### Definitions:

"exo-S-Mecamylamine" includes the d-enantiomer of N,2,3,3-tetramethylbicyclo-[2.1. 1]heptan-2-amine hydrochloride, 826-39-1. This enantiomer is also referred to as exo-S-N,2,3,3 -tetramethyl-bicyclo-[2.1.1]heptan-2-amine hydrochloride.

"Related exo-S-mecamylamine compounds" include various active stereoisomers and substituted analogs of mecamylamine (Stone et al., J Med Pharm Chem 5(4);665-90, 1962, hereby incorporated by reference). Activity can be tested in rats by nicotine convulsions, pupil dilatation and by other methods such as those described below. Such activity was routinely lost with larger substitutions for the methyl groups, which are not a part of this invention. Both methyl or dimethyl groups on the amino group were more active than other substituents and are included herein. The *d* form was active; however, the *dl* racemate appeared to be slightly more active. Consequently, the *l* form seems to have significant activity. Stone et al. reported that the *exo* form (methylamino group lies on the same plane as the methylene bridge) was always stronger than the *endo* form (methylamino group lies below the methylene bridge and tends to lie within the cage created by the bridge). In addition, a partial structure, 2,2,-dimethyl-3-methylaminobutane, also was active. Stone concluded that the slight differences in activity between different models for the *d* form and other analogs was not significant.

The term "substantially free of the exo-R-mecamylamine hydrochloride" as used herein means that the composition contains at least about 90% by weight of exo-S-mecamylamine - and less than about 10% by weight of exo-R-mecamylamine. In a more preferred embodiment, the composition contains at least 95% by weight of exo-S-mecamylamine and less than about 5% by weight of exo-R-mecamylamine. In the most preferred embodiment, the composition contains at least 99% by weight of exo-S-mecamylamine and less than about 1% by weight of exo-R-mecamylamine.

"Beneficial effect" is a noticeable improvement over the baseline clinically observable signs and symptoms and may include subjective patient reports of improvement. For example, a beneficial effect in motor disorders includes decreases in tic frequency or severity, but improvements also can be manifested indirectly through reductions in anxiety, aggressive outbursts, and premonitory urges that often precede or compound the severity of abnormal movements. Treatment effects can be quantified by clinical observations and videotape scoring. Beneficial effects can also be predicted by the results of animal screening. For example, Suemaru et al (*ibid*) has proposed that the nicotine-induced rat-tail tremor can be used to screen for compounds to treat tremors. Repeated nicotine administration can induce locomotor hyperactivity and a tail tremor in rats which is blocked with mecamylamine (0.1 - 1 mg/day, ip) but not by hexamethonium which does not readily enter the brain. (Suemaru K., Oishi R, Gomita Y, Arch Pharm 350:153-57, 1994).

The Yale Global Tic Severity Scale (YGTTS) is the most widely used clinical assessment rating scale used to assess tic symptoms. It provides an objective measure of tic frequency of severity based on clinical observations. This scale includes a tic symptom inventory which is filled out based on the patient's personal recall of tics occurring over the previous week. Using this inventory as a guide, the clinician then rates the severity of both motor and vocal tics on five separate dimensions: number, frequency, intensity, complexity, and interference. In addition, there is also a separate rating of global impairment which characterizes the impact of the disorder on the patient's social function, self esteem, etc., over the previous week.

An objective method for rating tic symptoms employs video recording of patients. A videotape of at least five minutes is viewed and the frequency and .severity of both motor and vocal tics are recorded. Video taping has proven a valuable adjunct to clinical rating systems for drug trials (Leckman JF, et al., Arch Gen Psychiatry, 48: 324-328, 1991; Shapiro ES, et al., Arch Gen Psychiatry, 46: 722-730; 1989; McConville BJ, Fogelson MH, Norman AB, Klykylo WM, Manderscheid MA, Parker KW, Sanberg PR, Am J Psychiatry, 148: 793-794, 1991; Silver AA, Shytle RD, Philipp MK, Sanberg PR, The Effects of Nicotine on Biological Systems 11. PBS Clarke, M. Quik and K. Thurau, (Eds.); Advances in Pharmacological Sciences, Birkhauser Publishers, pp. 293-299, 1995; Reveley MA, et al., Journal of Psychopharmacology Supplement, A30, 117, 1994)

Beneficial effects in obsessive compulsive disorders include diminution in the obsessive or compulsive behavior, which can be confirmed by patient or family reports. Beneficial effects in alcohol or cocaine abuse include longer drug-free periods as well as subjective feelings of less need for the drug.

"Side effects" are unwanted actions which may include but are not limited to cardiovascular effects, hypothermia, tremors, anti-diuresis, antinociception, blurred vision, impotency, dysuria, tremor, choreiform movements, mental aberrations, nervousness, depression, anxiety, insomnia, slurred speech, weakness, fatigue, sedation, headache, constipation, renal insufficiency, taste perversion (altered sense of taste), dizziness, and dyspepsia.

The term "effective amount" refers to the amount of exo-S-mecamylamine that is necessary to provide benefit. The precise amount required will vary depending upon the age and weight of the subject, severity of the disorder, route of administration, and so forth, but may easily be determined by routine experimentation, as described below in the clinical examples. In general, however, an effective amount of exo-S-mecamylamine range from about 0.001 mg/kg to about 6 mg/kg per day, preferably about 0.002 mg/kg to about 3 mg/kg, more preferably about 0.005 mg/kg to about 2 mg/kg, and most preferably about 0.01 to about 1.5 mg/kg. A starting dose for adults with drug-resistant TS is about 2.5 mg per day, with dosage adjusted according to return of symptoms. A small child with mild ADHD preferably starts with 1 mg per day or less.

The term "pharmaceutically acceptable" refers to a lack of unacceptable toxicity in a compound, such as a salt or excipient. Pharmaceutically acceptable salts include inorganic anions such as chloride, bromide, iodide, sulfate, sulfite, nitrate, nitrite, phosphate, and the like, and organic anions such as acetate, malonate, pyruvate, propionate, cinnamate, tosylate, citrate, and the like. Pharmaceutically acceptable excipients are described at length by E. W. Martin, in Remington's Pharmaceutical Sciences (Mack Publishing Co.).

Pharmaceutical compositions containing exo-S-mecamylamine may contain one or more pharmaceutical carriers. The term "pharmaceutically acceptable carrier" refers to any generally acceptable excipient that is relatively inert, non-toxic and non-irritating. When the carrier serves as a diluent, it may be solid, semisolid, or liquid material acting as a vehicle, excipient, or medium for the active ingredient. Pharmaceutical unit dosage forms may be prepared for administration by any of several routes, including, but not limited to, oral and parenteral (especially by intramuscular and intravenous injection, or by subcutaneous implant or transdermal administration). Representative of such forms are tablets, soft and hard gelatin capsules, powders, lozenges, chewing gums, emulsions, suspensions, syrups, solutions, sterile injectable solutions, and sterile packaged powders. Compositions containing nicotine antagonists may be formulated by procedures known in the art so as to provide rapid, sustained, or delayed release of any or all of the compounds after administration. In addition to the common dosage forms set out above, the compounds of the present invention may also be administered by controlled release means and/or delivery devices such as those described in U.S. Patent Nos. 3,845,770; 3,916,899; 3,536,809; 3,598,123; 4,008,719; 5,910,321; 5,348,746; and the like by the various manufacturers of controlled release means and/or delivery devices.

As the exo-S-mecamylamine formulation of the present invention is well suited to oral administration, preferred carriers facilitate formulation in tablet or capsule form. Solid pharmaceutical excipients such as magnesium stearate, calcium carbonate, silica, starch, sucrose, dextrose, polyethylene glycol (PEG), talc, and the like may be used with other conventional pharmaceutical adjuvants including fillers, lubricants, wetting agents, preserving agents, disintegrating agents, flavoring agents, and binders such as gelatin, gum arabic, cellulose, methylcellulose, and the like, to form admixtures which may be used as such or may be tabulated, encapsulated, or prepared in other suitable forms as noted above. A general description of formulation is given in Remington's Pharmaceutical Sciences (Mack Publishing Co.).

### Modes of Administration

Administration is preferably by oral dosage but may be by transdermal application, intranasal spray, bronchial inhalation, suppository, parenteral injection (e.g., intramuscular or intravenous injection), and the like. Carriers for parenteral administration include, without limitation, aqueous solutions of dextrose, mannitol, mannose, sorbitol, saline, pure water, ethanol, glycerol, propylene glycol, peanut oil, sesame oil, polyoxyethylene-polyoxypropylene block polymers, and the like. One may additionally include suitable preservatives, stabilizers, antioxidants, antimicrobials and buffering agents, for example, BHA, BHT, citric acid, ascorbic acid, tetracycline, and the like. Alternatively, one may incorporate or encapsulate the nicotine antagonist formulation in a suitable polymer matrix or membrane, thus providing a sustained-release delivery device suitable for implantation or application to the skin. Other devices include indwelling catheters and devices such as the Alzet® minipump.

The invention has been disclosed by direct description. The following are examples showing the efficacy of the method in providing benefit. The examples are only examples and should not be taken in any way as limiting to the scope of the method.

### Analysis of exo-S-Mecamylamine Hydrochloride

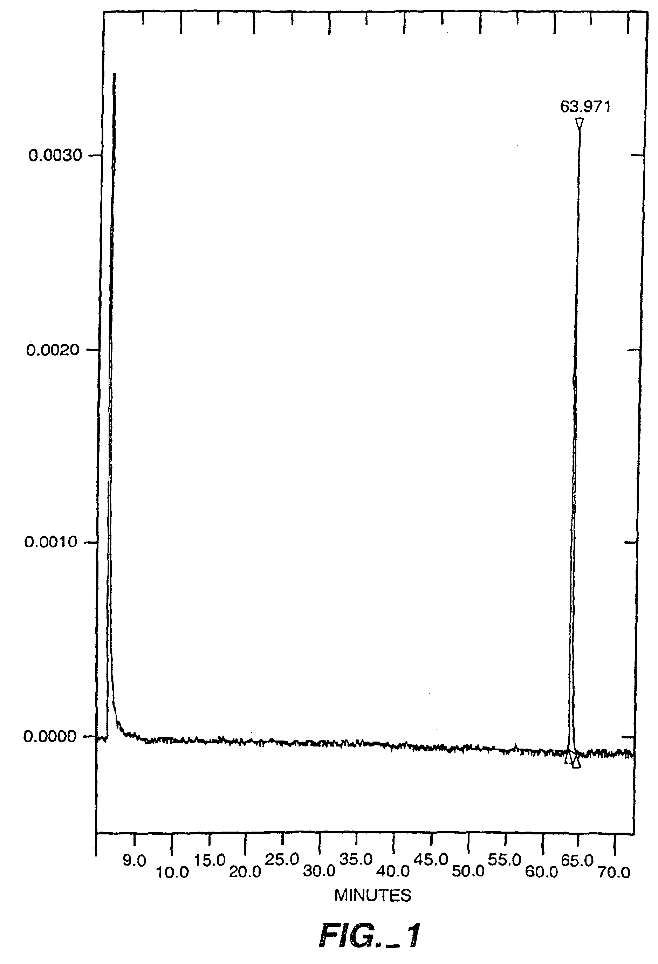

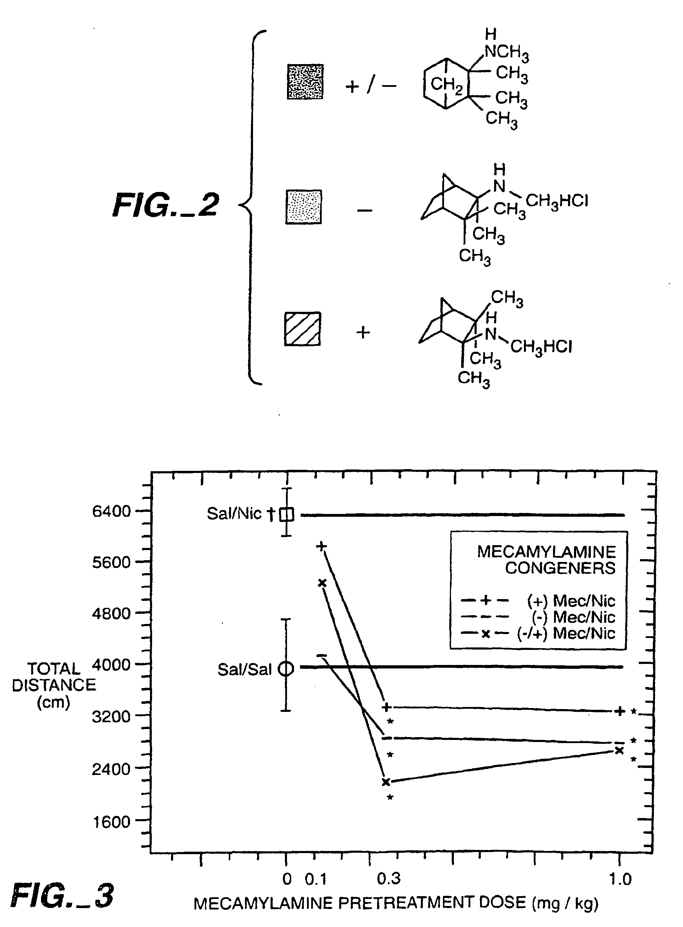

Mecamylamine chloride (Lot 2351) was 99.95% pure by gas chromatograph, as shown in Figure 1. The gas chromatograph retained exo-S-mecamylamine hydrochloride for 63.971 minutes, and no other significant peaks were observed. The chloride content was 17.2%, which was below the theoretical limit of 17.8% but within the specification. No camphene or other impurities were detected. Optical rotation was +19.4°. This lot was used in Examples 1 and 9 below. The structures of mecamylamine and the enantiomers are shown in Figure 2.

### Pharmacology

### General Methods

### Animals

Male Sprague-Dawley rats (Zici-Miller Laboratories, Allison Park, PA) weighing an average of 463 grams were used. They were housed in groups of 2-4 per cage, allowed free access to food and water, and maintained on a reverse 12h light/12h dark lighting cycle, with night being 8:00 AM through 8:00PM. All testing occurred during the rats' nocturnal cycle.

### Measurements and Apparatus

For all locomotor testing, a Digiscan Animal Activity Monitors (Model RXYSCM, Accuscan, Inc., Columbus, OH) was used. Box dimensions were 42 cm x 42 cm x 30 cm, and the walls and floors were clear acrylic. Each box used in this study had photocells that, when the light beam was broken by the rat's movement, calculate a number of variables. All locomotor activity was automatically captured and recorded with a Digipro software program.

To assess catalepsy (the ability to maintain position after being placed therein) induced by haloperidol and blocked with treatment, the bar test was used. The bar was placed 9 cm above the tabletop. The rat's forepaws were simultaneously placed on the bar and the hind paws placed under the rat for support. Time was measured from the second both forepaws were placed on the bar until the rat removed both paws from the bar. The minimum time was 1 second, and the maximum time allowed was 60 seconds. The shorter the time on the bar, the greater the blockage of haloperidol-induced catalepsy.

### Drugs

Mecamylamine HCl was obtained from Layton Bioscience, Inc., Atherton, CA. Optical isomers of mecamylamine were resolved from the racemate according to procedures reported by Stone et al (supra), but with significant modifications to improve optical purity and yields (see above). (-)-Nicotine was obtained from Sigma Chemical Co. (St. Louis, MO). Haloperidol lactate (Solopak®) was obtained from a local pharmacy. All drugs were dissolved in saline at a volume of I mg/ml and injected subcutaneously.

### Example 1

Eighty-eight experimentally naive adult male Sprague-Dawley derived rats were housed two per cage and allowed free access to food and water. Each rat received a randomly assigned pretreatment condition for seven consecutive days. On each day of this pretreatment period, rats received an injection of saline, racemic mecamylamine, exo-R-mecamylamine, or exo-S-mecamylamine 20 minutes prior to receiving a second injection of either saline or nicotine (0.4 mg/kg s.c.) and left in their home cage. Pretreatment assignment was arranged so that 2 rats from each condition were started and tested together to control for sequence effects. Rats received no treatment or testing on the day 8. On day 9, rats were tested for the presence of the sensitized locomotor stimulant response to nicotine. Each rat was placed into a locomotor box for a 60 minute habituation period, followed by a injection of nicotine (0.4 mg/kg s.c.), and then placed immediately back into the locomotor box. A computer recorded data over the next 60 minutes at 5-minute intervals.

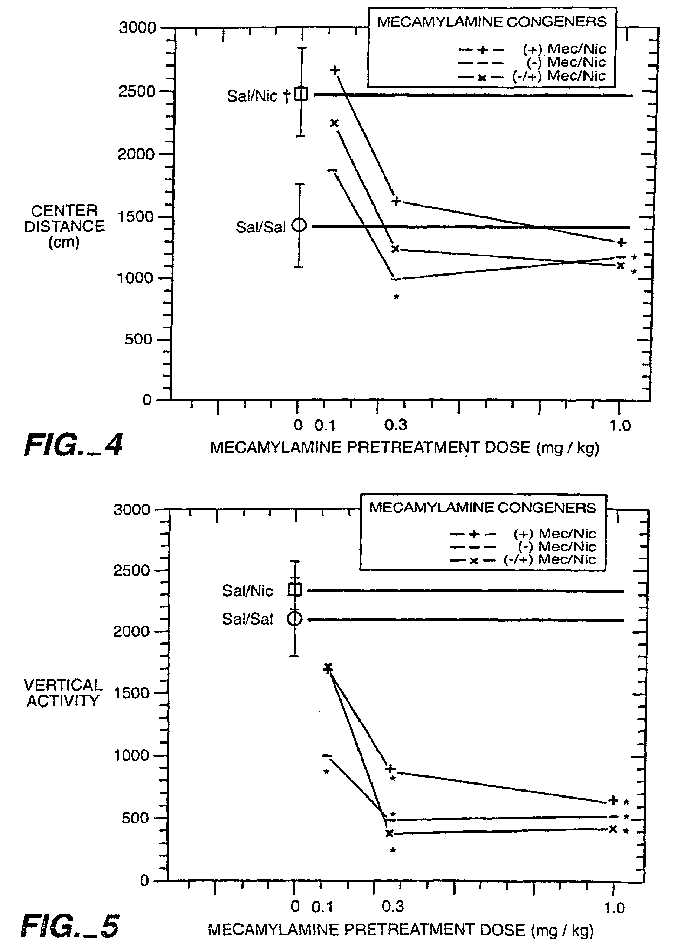

Figures 3-5 illustrate 3 dependent variables respectively for all groups following a test injection of 0.4 mg/kg nicotine on day 9. The saline/nicotine (sal/nic) pretreatment group exhibited a sensitized locomotor response to nicotine, which was not evident in any of the mecamylamine/nicotine (mec/nic) pretreatment groups. Further post-hoc comparisons indicated that the locomotor response to nicotine was significantly greater for the sal/nic pretreatment group when compared to the other groups (p<0.05). The response to nicotine in the mec/sal pretreatment groups was not significantly different from those receiving no nicotine in the sal/sal pretreatment group (p<0.05), except in the case of vertical activity, where all mec/sal groups had significantly less activity than control. At only 0.1 mg/kg, exo-R-mecamylamine effectively blocked the nicotine activity to control total distance.

Pretreatment with mecamylamine and both of its stereoisomers on nicotine exposure days, dose-dependently prevented the development of the sensitized locomotor responses to nicotine. Decreased vertical activity following the test injection of nicotine alone (day 9) was found in rats which had received chronic mecamylamine/nicotine exposure relative to those who had received chronic saline/saline exposure. This suggests that chronic exposure to mecamylamine actually reduce the locomotor response to nicotine to levels below that seen in the saline/saline group. Although both isomers of mecamylamine followed the same general pattern, exo-R-mecamylamine was generally more effective at lower doses, particularly for center distance and vertical activity. Interestingly, pre-exposure to exo-R-mecamylamine, but not exo-S-mecamylamine, prevented the expression of the sensitized nicotine response in center distance traveled. This suggests that exo-R-mecamylamine may be more effective than either the (+) isomer or racemic mecamylamine, in reducing the anxiolytic effects of nicotine in smokers.

### Example 2

This experiment was designed to determine if the enantiomers differ in their abilities to affect spontaneous locomotor activity. After a wash-out period of seven days, rats were randomly assigned to new groups of 8 rats each. Animals were injected with one of the following: saline, 3.0 mg/kg (+/-)-mecamylamine, 3.0 mg/kg exo-S-mecamylamine, or 3.0 mg/kg exo-R-mecamylamine. The rats were placed in the locomotor box for 60 min with data being collected at five-min intervals.

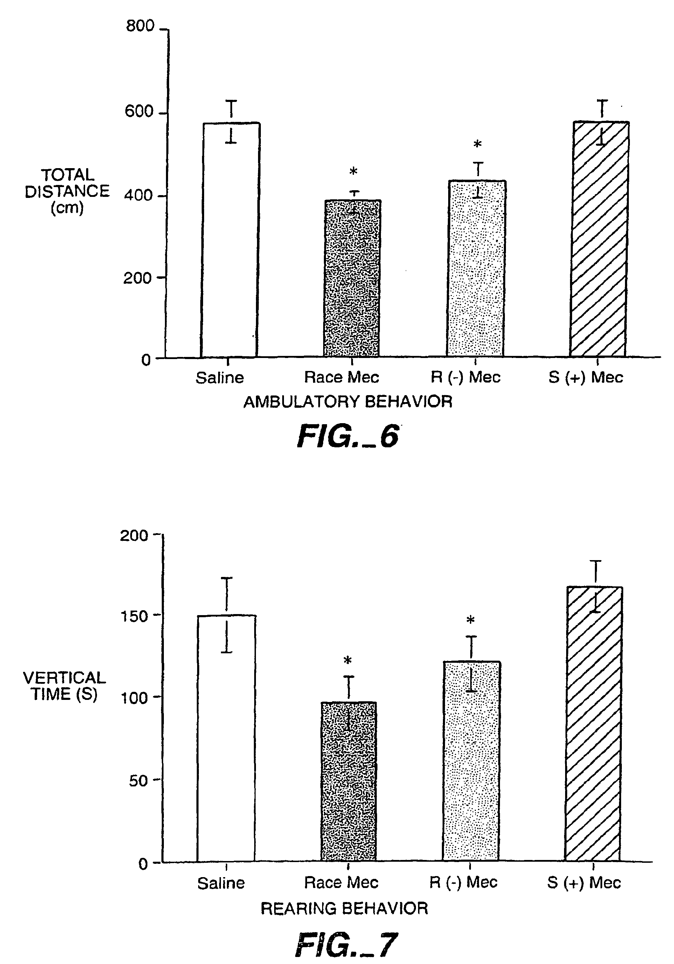

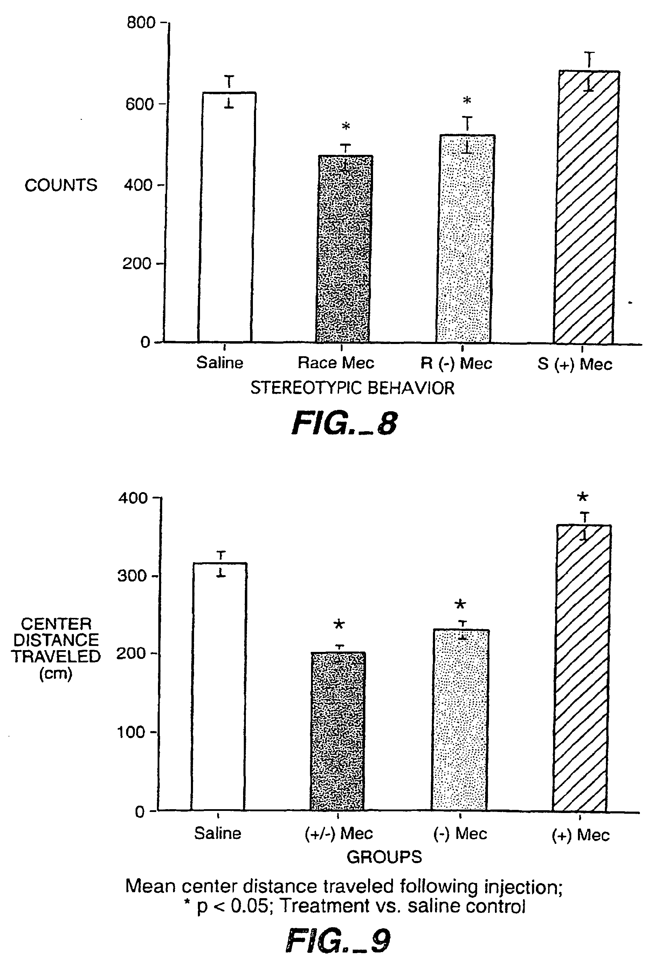

Figure 6 is the key to the next three figures. Figures 7, 8 and 9 show that racemic mecamylamine reduced spontaneous locomotor activity including total distance moved (Figure 6), vertical time (Figure 7), and stereotypic behavior (Figure 8). This pattern of reducing spontaneous locomotor activity was true of exo-R-mecamylamine as well. On the other hand, exo-S-mecamylamine produced essentially no effect on spontaneous locomotion, or in some cases, increased locomotor behavior. For example, rats receiving exo-S-mecamylamine exhibited significantly more locomotor activity in the center field than rats treated with saline (Figure 9).

When given alone, exo-R-mecamylamine tends to decrease spontaneous locomotion, while exo-S-mecamylamine either has no effect or actually increases locomotor behavior. For example, exo-S-mecamylamine significantly increased the distance traveled in the center of the open field. Since previous research with drugs which reduce anxiety in humans (e.g., Valium) also increase the distance traveled in the center of an open field, exo-S-mecamylamine may also reduce anxiety.

### Example 3

This experiment was designed to determine if the isomers differ in their duration of action in blocking the locomotor effects of nicotine. After a seven-day wash out period, rats were randomly assigned to two groups of eight rats each. All rats were then given 0.4 mg/kg nicotine s.c. injections once a day for five days. Each group then received the threshold dose of 0.3 mg/kg (-)-mecamylamine or 0.3/kg exo-S-mecamylamine at intervals of 1, 3, and 6 hr before receiving nicotine. On intervening days, rats in each group received saline at intervals of 1, 3, and 6 hr before receiving 0.4 mg/kg nicotine. Rats were allowed 30 min habituation in the locomotor box before receiving nicotine and then tested for 30 min.

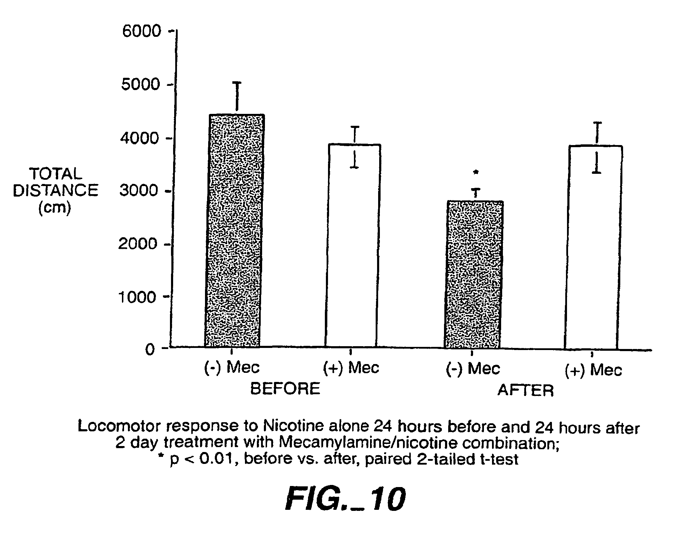

Figure 10 shows that rats pretreated with exo-R-mecamylamine two days in a row failed to exhibit a stimulant response to nicotine which was administered 24 hours after the last exo-R-mecamylamine dose (p<0.01, comparing before and after via the paired 2-tailed t-test). There was essentially no difference in stimulant response of the rats treated with exo-S-mecamylamine, This indicates that exo-R-mecamylamine has a longer duration of action (at least 24 hours in this test). Whether this effect involves pharmacokinetic or pharmacodynamic differences between the isomers has yet to be determined.

### Example 4

This experiment tested the effects of the mecamylamine enantiomers on haloperidol-induced catalepsy. 48 rats were randomly assigned to 4 groups of 12 rats each. This was a between-subjects design with each group of rats subjected to one of the following treatments: saline, 3,0 mg/kg (+/-)-mecamylamine, 3.0 mg/kg exo-S-mecamylamine or 3.0 mg/kg exo-R-mecamylamine. The rats received sc injections of 0.3 mg/kg of haloperidol 30 min prior to an sc injection of treatment. After an additional 30 min, rats were placed on the bar. Later, after a seven-day wash out period, rats received a saline sc injection 30 minutes prior to an sc injection of treatment drug and were placed on the bar 30 min after the injection. The experimenters were unaware of the rat's treatment, and the same experimenter administered the test each time.

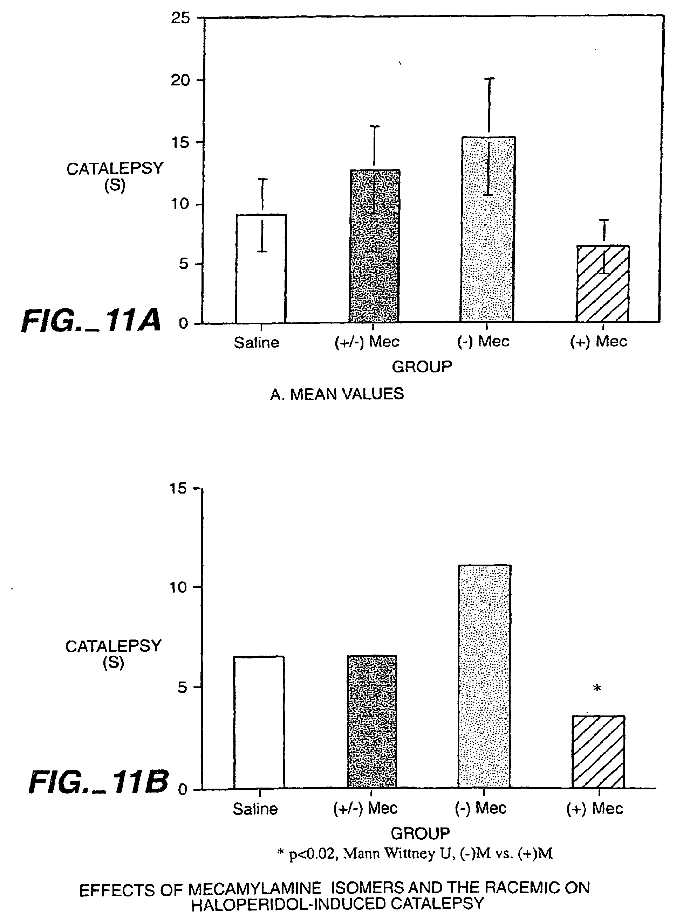

Figures 11A and 11B shows that exo-R-mecamylamine tended to increase haloperidol-induced catalepsy, while exo-S-mecamylamine tended to reduce the cataleptic response to haloperidol. This finding suggests the exo-R-mecamylamine may be useful for hyperkinetic movement disorders, while exo-S-mecamylamine may be useful for hypokinetic movement disorders.

### Example 5

Recently it has been shown that some seizure disorders, including but not limited to juvenile myoclonic epilepsy, autosomal dominant nocturnal frontal lobe epilepsy and possibly inherited idiopathic epilepsy, are mediated through the same receptors that bind nicotine in the brain. Nicotine has been shown to induce short periods of seizure activity in rats. Okamoto et al. (Jpn J Pharmacol 59:449-55, 1992) showed that a single high dose of mecamylamine (1.0 mg/kg) blocked nicotine-induced seizures in rats. The present experiment evaluates the effect of exo-R-mecamylamine in blocking nicotine-induced seizures in rats. The dose for exo-R-mecamylamine ranges from 0.1 to 3.0 mg/kg/day and for nicotine from 2.5 to 5 mg/kg/day. Administration is by the intraperitoneal or other feasible route. An acute ip injection of exo-R-mecamylamine or saline is given 15 minutes prior to administration of nicotine. After drug administration, each rat is placed in an observation test box for 30 minutes, and seizure activity is recorded.

### Example 6

The behavioral effects of the dopamine agonist apomorphine has provided a useful animal model for hyperdopaminergic disorders such as Tourette's Syndrome. When administered to rats, apomorphine induces stereotypic movement and licking behavior. In a dose-dependent manner, nicotine alters (increased at doses of 0.05 and 0.5 µg/kg and decreased at 250 µg/kg) the licking behavior when it is administered before apomorphine. Mecamylamine (1 and 3 mg/kg) decreased the response to nicotine and increased spontaneous grooming (Zarrindast et al. J Psychopharmacol 12:375-9, 1998). Mecamylamine (0.05, 0.25 and 0.5 mg/kg ip) profoundly reduced the rat licking response to apomorphine (Zarrindast et al. Eur Neuropsychopharmacol 9:235-8, 1999). The exo-S-mecamylamine enantiomer is tested for its ability to block the stereotypic response to apomorphine in rats. The route of administration is sc or ip injection. Doses for exo-S-mecamylamine are about 0.1-3.0 mg/kg/day, for apomorphine 0.5-2.0 mg/kg/day and for nicotine 0.4 mg/kg/day. In the acute test, each rat receives saline or exo-S-mecamylamine 15 minutes prior to receiving apomorphine or nicotine. Immediately following the second injection, each rat is placed in a locomotor box for one hr of testing. Rats may be used in a chronic study and receive similar treatment, except that their pretreatment entails 7 days of exposure to saline or exo-S-mecamylamine.

### Example 7

This experiment evaluates the effect of exo-S-mecamylamine on nicotinic receptors involved in the neuroendocrine response to stress. This experiment uses acute stress caused by brief exposure to a cat. A low dose (0.1 mg/kg) of racemic mecamylamine prevents the neuroendocrine response to cat exposure stress (Shytle et al. Soc Neurosci Abstr 24:371-15, 1998). The rats are pretreated with saline or exo-S-mecamylamine injected sc. Doses for exo-S-mecamylamine are about 0.01-3.0 mg/kg/day. Next, the rats are placed in a circular, clear Plexiglas container that is divided into 8 section, one for each rat. The cat is then placed on top of the container for 20 minutes. No-stress controls are placed in their home cage. After 20 min, the rats are removed and immediately decapitated for neurochemical assays. Blood is collected from each rat to measure plasma corticosterone levels. The brain of each rat is removed for assays of CRF and catecholamine levels.

### Example 8

The antihypertensive effects of the exo-S-mecamylamine enantiomer are demonstrated by measuring the blockade of the pressor response elicited by sympathetic nerve stimulation in the pithed rat. Rats are anesthetized with halothane (2% in O₂), the right carotid artery and jugular vein are cannulated, and both vagal nerves are cut at the mid-cervical level. The left carotid artery and jugular vein are tied off to reduce cerebral blood supply further. The arterial line is attached to a pressure transducer for continuous recording of systemic blood pressure and heart rate. The venous line is used for compound injection. Rats are pithed by inserting a steel rod through the orbit and foramen magnum down the spinal cord to the first sacral vertebra; this rod is also used to deliver electrical stimulation to the sympathetic outflow. Immediately after the animals are pithed, artificial respiration is instituted with O₂-enriched air, an indifferent electrode is inserted under the skin of the back, and gallamine is administered (20 mg/kg, iv) to prevent muscle contractions. Thirty minutes rest is allowed to stabilize cardiovascular parameters. Stimulation-evoked pressor responses before and after exo-S-mecamylamine treatment are measured by evoking sympathetic outflow at 1 Hz, 40 V, 1 ms pulse duration while cardiovascular parameters are monitored continuously. Reductions in stimulation-evoked elevations in pulse pressure reflect sympathoinhibition by exo-S-mecamylamine. If the pressure does not rise, that indicates that the drug has a sympatholytic effect and is a candidate for further antihypertensive testing. Alternately, the test is predictive for orthostatic hypotension as a side effect.

### Example 9

This experiment evaluates the efficacy and potency of exo-S-mecamylamine on human α₃β₄, α₄β₂, α₃β₂, and α₇ receptors expressed in *Xenopus* oocytes and compares its activity to that of mecamylamine. Voltage dependence and binding reversibility also are determined. Mature female *Xenopus laevis* African toads are used as a source of oocytes. After linearization and purification of cloned cDNAs, RNA transcripts are prepared in vitro using the appropriate mMessage mMachine® kit from Ambion Inc. (Austin TX). Harvested oocytes are treated with collagenase (Worthington Biochemical Corporation, Freehold NJ) for t hr at room temperature in calcium-free solution. Subsequently stage 5 oocytes are isolated and injected with 50 nL each of a mixture of the appropriate subunit(s) cRNAs. Recordings are made about 1-7 days after cRNA injection.

For electrophysiology, oocyte recordings are made with an oocyte amplifier (e.g., Warner Instruments, Hamden, CT, No. OC-725C) and recording chamber. Oocytes are placed in the recording chamber with a total volume of about 0.6 ml and perfused at room temperature by frog Ringer's solution (115 mM NaCl, 2.5 mM KCl, 10 mM HEPES pH 7.3, and 1.8 mM CaCl₂) containing 1 uM atropine to inhibit potential muscarinic responses. A Mariotte flask filled with Ringer's solution is used to maintain a constant hydrostatic pressure for drug delivery and washes. Drugs are diluted in perfusion solution and loaded into a 2 ml loop at the terminus of the perfusion line. A bypass of the drug-loading loop allows bath solution to flow continuously while the drug loop is loaded. The drug application is synchronized with data acquisition by using a 2-way electronic valve. The rate of bath solution exchange and drug application is preferably about 6 ml/min. Current electrodes are filled with a solution containing 250 mMCsCl, 250 mM Csf and 100 mM EGTA and have resistances of 0.5-2 MΩ. Voltage electrodes are filled with 3M KCl and have resistances of 1-3MΩ. Oocytes with resting membrane potentials more positive than -30 mV are not used.

Measurements of current responses to exo-S-mecamylamine application are studied under two-electrode voltage clamp. Holding currents immediately prior to exo-S-mecamylamine application are subtracted from measurements of the peak response to drug. All drug applications are separated by at least a 5 min wash period, longer if there is persisting drug effect. At the start of recording, all oocytes receive two initial control applications of ACh. The second application of control ACh minimizes the effects of rundown that occasionally occur after an initial ACh-response. The second application of ACh also is used to normalize for the level of channel expression in each oocyte. To determine residual inhibitory effects, application of ACh with inhibitor or inhibitor alone is followed by another application of ACh alone and compared to the pre-application control ACh response.

For each receptor subtype, a control ACh concentration is selected that is sufficient to stimulate the receptors to a level representing a reasonably high value of Pₒₚₑₙ at the peak of the response while minimizing rundown from successive ACh applications. Such conditions are adequate to achieve maximal inhibition. The control ACh concentration for α₃-containing receptors is typically about 100 µM and for α₁β₂ receptors 10 µM, because higher ACh concentrations inhibit the maximum response obtainable.

For experiments assessing voltage-dependence of drug inhibition, oocytes are initially voltage clamped at a holding potential of -50 mV, and a control application of ACh alone is delivered. A second control response is then obtained at the designated test potential. The holding potential is kept at the designated voltage for the co-application of ACh with exo-S-mecamylamine. Residual inhibition is evaluated with a subsequent application of ACh alone at the test potential, after a 5-min wash period.

For oocytes with the α3β4 receptors, racemic and exo-S-mecamylamine had similar normalized dose response curves and IC50, from which it can be deduced that exo-R-mecamylamine also would be similar. However, the racemic and exo-S-mecamylamine dose response curves after a 5-min wash were different, with exo-S-mecamylamine eliciting a higher response at most doses. This suggests that exo-S-mecamylamine has a less persistent block of the receptor and that the R-enantiomer is responsible for longer activity with this important peripheral receptor. The IC50 for the S-enantiomer was almost twice as great at that of the racemate.

When previously exposed oocytes with the α₄β₂ receptor were washed, allowed to recover, and treated with racemic mecamylamine, there was a biphasic curve, which suggests a biphasic dissociation of the racemate for this receptor, too. About 50% of the inhibition is gone in 7-8 minutes and the remaining 50% takes much longer, estimated at 5-10 times as long. Exo-R-mecamylamine hydrochloride may have a very long half life at this important central nervous system receptor.

In summary, these experiments shows important pharmacological differences in the actions of exo-R-mecamylamine and exo-S-mecamylamine. These isomers are effective in the treatment of the following indications (but are not limited thereto): Tourette syndrome, mood disorders (e.g., bipolar disorder and depression), anxiety disorders, tremor, alcoholism, opiate and amphetamine addiction, seizure disorders, and nicotine responsive disorders (e.g., schizophrenia, Parkinson's disease, and attention deficit hyperactivity disorder), substances of abuse, such as cocaine and alcohol. This discovery indicates that both isomers act as potent nicotine antagonists, while avoiding the usual adverse effects associated with the racemic mixture of mecamylamine hydrochloride.

### Other Uses

Recent reports suggest that nicotine reduces the symptoms of schizophrenia (Adler LE et al, Am J Psychiatry 150: 1856-1861, 1993), Attention Deficit Hyperactivity Disorder (ADHD) (Levin ED et al, Psychopharmacology 123: 55-62, 1995) and depression (Salin-Pascual RJ et al, Psychopharmacology 121(4): 476-479, 1995). While it is generally believed that nAChr activation is responsible for nicotine's therapeutic actions in these "nicotine-responsive" disorders (Decker MW et al, Life Sci, 56: 545-570, 1995), it is clear that, like many other drugs, nicotine has complex neuropharmacological effects. Thus, many people with such nicotine-responsive disorders, could be helped with a nAChr blocker which has been disclosed herein with the example of mecamylamine, a nAChr blocker, which reduced the symptoms in the nicotine responsive disorders, TS and ADHD.

Schizophrenia, a psychiatric disorder theorized to involve hyperdopaminergic tone, is most often treated with neuroleptics; but there is now speculation that it is a nicotine-responsive disorder. For example, surveys of schizophrenic patients have demonstrated rates of smoking between 74% and 92%, compared to 35% to 54% for all psychiatric patients and 30%-35% for the general population. It has been speculated that cigarette smoking may improve underlying psychopathology by enhancing concentration and reducing anxiety from hyperarousal (Gopalaswamy AK, Morgan R, Br J Psychiatry, 149: 523, 1986). In addition, nicotine may have some role to play in reducing the cognitive deficits associated with schizophrenia and neuroleptic treatment. Cigarette smoking has been found to normalize sensory gating deficits in schizophrenic patients (Adler LE et al, Am J Psychiatry 150:1856-1861, 1993) and a recent study found that transdermal nicotine reversed some of the adverse cognitive effects of standard anti-psychotic medication and improved cognitive performance in general for schizophrenic patients (Levin ED et al, Psychopharmacology 123:55-63, 1996). If as we now hypothesize that nicotine administration may actually have a similar effect as a nAChr blocker, then it is possible that a nAChr blocker such as a mecamylamine isomer would also reverse the adverse cognitive effects of the anti-psychotic medication and improve cognitive performance in schizophrenic patients. Moreover, since nicotine potentiates the therapeutic effects of neuroleptics in TS (McConville BJ et al, Biological Psychiatry 31: 832-840, 1992), the use of mecamylamine as an adjunct to neuroleptics in "neuroleptic-responsive" disorders such as schizophrenia and Huntington's chorea, can allow for reducing the neuroleptic dose, thereby reducing the side effects of the neuroleptic without reducing its therapeutic effects.

Cocaine use is an increasingly common problem in the United States, with estimates of lifetime use prevalence rates at 2.5% and current prevalence rates of cocaine abuse or dependence rates of about I %. (Regier et al., 1990). There are no known effective treatments, aside from expensive, personnel-intensive supervision and counseling programs.

Many schizophrenic and depressed patients also have a high incidence of cocaine use; rates are estimated to be 40-50% (Shaner et al., 1995). Of cocaine abusers, it has been estimated that as many as 75% also are dependent on nicotine (Budney et al., 1993), as opposed to a smoking rate of 22% in controls.

Animal results with regard to cocaine, nicotine and mecamylamine are equivocal. On the one hand, cocaine and its analogues bind calf brain with modest affinity to the non-competitive ion channel site on the high-affinity nAChR, the site of action of mecamylamine (Lerner-Marmarosh N, Carroll FI and Abood LG, Life Sciences 56(3): 67-70, 1995). Cocaine was moderately effective in antagonizing the behavioral effects of nicotine. However, in mice, systemic administration of mecamylamine (1 mg/kg) and dihydro-beta-erythroidine (2 mg/kg) - nicotinic antagonists - and atropine (2 mg/kg) - a muscarinic antagonist - were ineffective against psychostimulant-induced stereotypy in naive animals. All three drugs were ineffective against either the induction or expression of cocaine sensitization. Karler, Brain Res. 1996 (Jul 1) 725(2): 192-8. Spealman and Goldberg tested the effects of mecamylamine on the schedule-controlled behavior by intravenous injections of nicotine and cocaine in squirrel monkeys. J Pharm Exp Therap 223: 402-06, 1982. Administering mecamylamine before the experimental session causing responding maintained by nicotine, but not by cocaine, to fall within saline-control levels. Nevertheless, based on the above experiences of mecamylamine in Tourette's, bipolar patients and patients with schizophrenia-like symptoms, cocaine abusers are also likely to benefit from treatment with mecamylamine and other nicotine antagonists.

The treatment of viral infections, particularly herpes I and II, has been successfully undertaken with ganglionic blocking agents tetraethylammonium ion or hexamethonium ions (U.S. Patent No. 5,686,448). Because exo-S-mecamylamine has ganglionic blocking action, it can be expected to be similarly efficacious against viral infections.

Mecamylamine has been shown to reduce organophosphate poisoning toxicity. For example, when rats were dosed with 8 mg/kg of DFP (an organophosphate), all died within 5 hours. However, 3 of 4 rats receiving mecamylamine at 30 mg/kg and the lethal dose of DFP survived beyond 5 hours. Rats receiving a combination of mecamylamine and 2-PAM and then the lethal dose of DPP all survived. It would be beneficial to lower the dose of mecamylamine by administering only the effective isomer.

Alpha₄, but not alpha₃ and alpha₇, nicotinic acetylcholine receptor subunits are lost from the temporal cortex in Alzheimer's disease. Neuronal nicotinic acetylcholine receptors labelled with tritiated agonists are reduced in the cerebral cortex in Alzheimer's disease (AD). Autopsy tissue from the temporal cortex of 14 AD cases and 15 age-matched control subjects was compared using immunoblotting with antibodies against recombinant peptides specific for alpha₃, alpha₄, and alpha₇ subunits, in conjunction with [3H]epibatidine binding. Antibodies to alpha₃, alpha₄, and alpha₇ produced one major band on western blots at 59, 51, and 57 kDa, respectively. [3H]Epibatidine binding and alpha₄-like immunoreactivity (using antibodies against the extracellular domain and cytoplasmic loop of the alpha₄ subunit) were reduced in AD cases compared with control subjects (p < 0.02) and with a subgroup of control subjects (n = 9) who did not smoke prior to death (p < 0.05) for the former two parameters. [3H]Epibatidine binding and cytoplasmic alpha₄-like immunoreactivity were significantly elevated in a subgroup of control subjects (n = 4) who had smoked prior to death (p < 0.05). There were no significant changes in alpha₃- or alplia₇-like immunoreactivity associated with AD or tobacco use. The selective involvement of alpha₄ has implications for understanding the role of nicotinic receptors in AD and potential therapeutic targets (Martin-Ruiz CM et al. *Neurochem* 1999 Oct;73(4):1 b35-40).

The foregoing description and examples are intended only to illustrate, not limit, the disclosed invention.

## Claims

1. Use of exo-S-mecamylamine or a pharmaceutically acceptable salt thereof, substantially free of exo-R-mecamylamine in the manufacture of a medicament for the treatment of neuropsychiatric disorders in a human.

2. The use of claim 1 wherein said exo-S-mecamylamine or salt is used in an amount of from 0.5 mg to 20 mg.

3. The use of claim 1 or 2 wherein said medicament comprises exo-S-mecamylamine hydrochloride.

4. The use of claim 1, 2 or 3 wherein said medicament is adapted for use in oral administration, intravenous administration, a transdermal formulation, a transdermal patch, a solid preparation, or a sustained release formulation.

5. The use of claim 4 wherein said medicament is adapted for use in a sustained release formulation and in a tablet, capsule or gelcap.

6. The use of any one of the preceding claims wherein:
(a) the substantially pure exo-S-mecamylamine is greater than 95% by weight and exo-R-mecamylamine is less than 5% by weight;
(b) the substantially pure exo-S-mecamylamine is greater than 99% by weight and exo-R-mecamylamine is less than 1% by weight;
(c) the substantially pure exo-S-mecamylamine is greater than 99.5% by weight and exo-R-mecamylamine is less than 0.5% by weight; or
(d) the substantially pure exo-S-mecamylamine is greater than 99.7% by weight and exo-R-mecamylamine is less than 0.3% by weight.

7. A use according to any one of the preceding claims wherein said neuropsychiatric disorder is bipolar disorder, depression, an anxiety disorder, schizophrenia, a seizure disorder, Parkinson's disease, attention deficit hyperactivity disorder, Tourette's syndrome, obsessive compulsive disorder, hemidystonia, Huntingdon's disease, cocaine addiction, alcohol addiction, amphetamine addiction or opiate addiction.

8. A use according to claim 7 wherein said medicament is adapted for administration intravenously, transdermally, intrathecally, orally or by bolus injection.

9. A use according to claim 7 or 8 wherein said medicament is suitable for administration one to four times per day.

## Patentansprüche

1. Verwendung von exo-S-Mecamylamin oder eines pharmazeutisch annehmbaren Salzes davon, im Wesentlichen frei von exo-R-Mecamylamin, bei der Herstellung eines Medikamentes zur Behandlung neuropsychiatrischer Störungen im Menschen.

2. Verwendung nach Anspruch 1, worin das exo-S-Mecamylamin oder Salz in einer Menge von 0,5 mg bis 20 mg verwendet wird.

3. Verwendung nach Anspruch 1 oder 2, worin das Medikament exo-S-Mecamylamin Hydrochlorid umfasst.

4. Verwendung nach Anspruch 1, 2 oder 3 worin das Medikament zur Verwendung bei oraler Verabreichung, intravenöser Verabreichung, in einer transdermalen Formulierung, einem transdermalen Pflaster, einer festen Zubereitung oder einer verzögert freisetzenden Formulierung angepasst ist.

5. Verwendung nach Anspruch 4, worin das Medikament für die Verwendung in einer verzögert freisetzenden Formulierung und in einer Tablette, Kapsel oder einer Gelkapsel angepasst ist.

6. Verwendung nach einem der vorstehenden Ansprüche, worin
(a) das im Wesentlichen reine exo-S-Mecamylamin mehr als 95 Gew.-% und das exo-R-Mecamylamin weniger als 5 Gew.-% ausmacht;
(b) das im Wesentlichen reine exo-S-Mecamylamin mehr als 99 Gew.-% und das exo-R-Mecamylamin weniger als 1 Gew.-% ausmacht;
(c) das im Wesentlichen reine exo-S-Mecamylamin mehr als 99,5 Gew.-% und das exo-R-Mecamylamin weniger als 0,5 Gew.-% ausmacht; oder
(d) das im Wesentlichen reine exo-S-Mecamylamin mehr als 99,7 Gew.-% und das exo-R-Mecamylamin weniger als 0,3 Gew.-% ausmacht.

7. Verwendung nach einem der vorstehenden Ansprüche, worin die neuropsychiatrische Störung eine manisch depressive Psychose, Depression, eine Angstneurose, Schizophrenie, eine Krampfanfall auslösende Störung, die Parkinsonsche Erkrankung, das hyperkinetische Syndrom des Kindesalters (ADS), das Tourette-Syndrom, eine obsessiv-kompulsive Reaktion, die Hämidystonie, die Corea-Huntingdon eine Kokainabhängigkeit, eine Alkoholabhängigkeit, eine Amphetaminabhängigkeit oder eine Opiatabhängigkeit ist.

8. Verwendung nach Anspruch 7, worin das Medikament an die intravenöse, transdermale, intrathekale, orale Verabreichung oder an die Verabreichung mittels Bolusinjektion angepasst ist.

9. Verwendung nach Anspruch 7 oder 8, worin das Medikament für die Verabreichung 1 bis 4 mal täglich geeignet ist.

## Revendications

1. Utilisation de l'exo-S-mécamylamine ou d'un sel pharmaceutiquement acceptable de celle-ci, substantiellement libre d'exo-R-mécamylamine, dans la fabrication d'un médicament pour le traitement de troubles neuropsychiatriques chez un humain.

2. Utilisation selon la revendication 1, dans laquelle l'exo-S-mécamylamine ou ledit sel est utilisé(e) en une quantité de 0,5 mg à 20 mg.

3. Utilisation selon la revendication 1 ou 2, dans laquelle ledit médicament comprend du chlorhydrate d'exo-S-mécamylamine.

4. Utilisation selon la revendication 1, 2 ou 3, dans laquelle ledit médicament est adapté à une administration par voie orale, par voie intraveineuse, par voie transdermique, ou par voie transdermique au moyen d'un timbre, ou est adapté à une préparation solide ou à celle d'une formulation à libération prolongée.

5. Utilisation selon la revendication 4, dans laquelle ledit médicament est adapté à une utilisation selon une formulation à libération prolongée, un comprimé, une capsule ou une gélule.

6. Utilisation selon l'une quelconque des revendications précédentes, dans laquelle :
a) l'exo-S-mécamylamine substantiellement pure est à plus de 95 % en poids et l'exo-R-mécamylamine est à moins de 5 % en poids ;
b) l'exo-S-mécamylamine substantiellement pure est à plus de 99 % en poids et l'exo-R-mécamylamine est à moins de 1 % en poids ;
c) l'exo-S-mécamylamine substantiellement pure est à plus de 99,5 % en poids et l'exo-R-mécamylamine est à moins de 0,5 % en poids ; ou
d) l'exo-S-mécamylamine substantiellement pure est à plus de 99,7 % en poids et l'exo-R-mécamylamine est à moins de 0,3 % en poids.

7. Utilisation selon l'une quelconque des revendications précédentes, dans laquelle ledit trouble neuropsychiatrique est la psychose maniacodépressive, la dépression, une crise d'angoisse, la schizophrénie, la crise d'épilepsie, la maladie de Parkinson, le trouble de l'hyperactivité avec déficit de l'attention, le syndrome de Tourette, le trouble obsessionnel compulsif, l'hémidystonie, la maladie de Huntingdon, la cocaïnomanie, l'alcoolisme, la dépendance à l'amphétamine ou la toxicomanie opiacée.

8. Utilisation selon la revendication 7, dans laquelle ledit médicament est adapté à une administration par voie intraveineuse, par voie transdermique, par voie intrathécale, par voie orale ou par injection en bolus.

9. Utilisation selon la revendication 7 ou 8, dans laquelle ledit médicament est approprié pour une administration une à quatre fois par jour.
